# EUROPÄISCHE PATENTANMELDUNG

(11) **EP 0 839 828 A1**
(43) Veröffentlichungstag der Anmeldung: **06.05.1998**
(21) Anmeldenummer: 97116482.7
(22) Anmeldetag: 22.09.1997
(51) Int. Cl.: C07H 19/10, C07H 19/20, C07K 14/00, C07H 21/00

(54) **Bausteine für DNA/PNA-Cooligomere**

(30) Priorität: 04.10.1996 DE 19640974
(71) Anmelder: BAYER AG, 51368 Leverkusen (DE)
(72) Erfinder: Jordan, Stephan, Dr., 51061 Köln (DE); Schwemler, Christoph, Dr., 42799 Leichlingen (DE)

(57) **Zusammenfassung**

Die Erfindung betrifft Verbindungen der allgemeinen Formel (I) 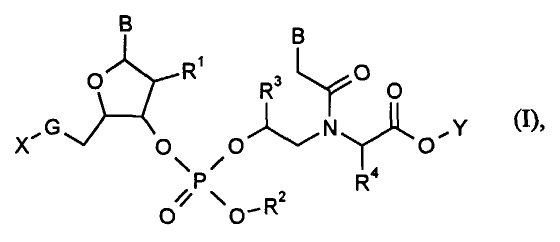 in welcher die Substituenten die in der Beschreibung angegebenen Bedeutungen haben.

Die Verbindungen eingen sich zur Herstellung von bisher unbekannten streng alternierender PNA/DNA Cooligomere.

## Beschreibung

Peptidnukleinsäuren (PNA) sind polyamidische Verbindungen, die in der Seitenkette Purine oder Pyrimidine tragen. Es handelt sich somit formal um DNA-analoge Verbindungen, bei denen das Desoxyribosephosphatgetüst durch ein polyamidisches Rückgrat ersetzt wurde.

Das gezielte Abschalten von Genexpression durch komplementäre Nukleinsäuren, sogenannte Antisense-Oligonukleotide, stellt einen neuen Therapieansatz dar. Mögliche Anwendungen reichen von der Behandlung viraler Infektionen bis zur Therapie von Krebs (S. Agrawal, Tibtech 10, 152 (1992); W. James, Antiviral Chemistry & Chemotherapy 2, 191 (1991); B. Calabretta, Cancer Research 51, 4504 (1991)). Die Kontrolle der Genexpression erfolgt auf der Ebene von DNA und RNA und gelingt bereits mit unmodifizierten Oligonukleotiden (C. Helene, Anti-Cancer Drug Design 6, 569 (1991); E. Uhlmann, A. Peymann, Chemical Reviews 90, 543 (1990)). Diese sind jedoch aufgrund mangelnder enzymatischer Stabilität und zu geringer Aufnahme in zelluläre Systeme für therapeutische Anwendungen nicht geeignet. Therapeutische Anwendungen erfordern chemisch modifizierte Antisense-Oligonukleotide.

Neben der Antisense-Strategie kann eine Inhibition der Genexpression auch durch die Sense-Strategie erzielt werden. Dabei konkurrieren die Sense-Oligonukleotide spezifisch mit DNA-Bindungsproteinen wie z.B. Transkriptionsfaktoren (M. Blumengeld, Nucleic Acids Research 21, 3405 (1993)).

Oligonukleotide mit modifiziertem Internukleotidphosphat oder einer phosphatfreien Internukleotidverknüpfung wurden in vielen Arbeiten systematisch untersucht; ihre Synthese erwies sich jedoch als sehr aufwendig und beobachtete therapeutische Effekte als nicht ausreichend (E. Uhlmann, A. Peyman, Chemical Reviews 90, 543 (1990)).

Eine Alternative zur Modifikation oder Substitution der Phosphatgruppe in Nukleinsäuren ist der komplette Austausch von Ribose und Phosphat durch andere Rückgrate. Dieses Konzept wurde erstmals von Pitha et al. realisiert, der Ribosephosphat durch Poly-N-Vinyl-derivate ersetzte, was zu sogenannter "Plastik-DNA" führt (J. Pitha, P.O.P. Ts'O, J. Org. Chem. 33, 1341 (1968); J. Pitha, J. Adv. Polym. Sci. 50, 1 (1983)). Es erlaubt jedoch nicht den gezielten Aufbau definierter Sequenzen.

Die Synthese definierter Sequenzen gelingt, wenn anstelle von Zuckerphosphat beispielsweise ein Polyamid-Rückgrat verwendet wird, das in Analogie zur konventionellen Peptidsynthese (M. Bodanszky, Principles of Peptide Synthesis, Springer, Berlin 1984) schrittweise aufgebaut wird. Dieses Konzept wurde von verschiedenen Arbeitsgruppen unterschiedlich realisiert (J.E. Summerton et al. WO 86/05518; R. S. Varma et al. WO 92/18518; O. Buchardt et al. WO 92/20702; H. Wang, D. D. Weller, Tetrahedron Letters 32, 7385 (1991); P. Garner, J. U. Yoo, Tetrahedron Letters 34, 1275 (1993); S.-B. Huang, J. S. Nelson D. D. Weller, J. Org. Chem 56, 6007 (1991); Bayer AG EP 646 595 A1, EP 646 596 A1 u. EP 700 928 A1; Hoechst AG EP 672 661 A1, EP 672 677 A2, EP 672 700 A1).

Polyamid-Nukleinsäuren eignen sich ebenfalls für diagnostische und molekularbiologische Anwendungen (Buchardt et al. WO 92/20703 und Glaxo Inc. WO 93/12129).

Nachdem sich Oligomere aus reinen PNA-Bausteinen als zu lipophil und damit unfähig zur Zellwandpenetration erwiesen haben, konnte dieses Problem durch Kombinationen von "hydroxysubstiuierten" PNA-Monomeren mit 5'-Amino-2',5'-didesoxynucleotiden oder 2'-Desoxynucleotiden zu DNA/PNA-Cooligomeren reduziert werden (vgl. Hoechst AG EP 672 677 A2). Heterooligomere Verbindungen mit einem Rückgrat aus 2-Aminoethyl-glycin und L-trans-Aminoprolin haben sich in Bezug auf Bindungsstärke zur komplementären DNA gegenüber Verbindungen mit reinem 2-Aminoethylglycin-Rückgrat als überlegen erwiesen (vgl. EP 700 928 A1). Intention der vorliegenden Erfindung ist es, durch Ersatz des 2-Aminoethylglycins durch Nucleotidderivate und des Aminoprolins durch 2-Hydroxyethylglycin-Analoga zu Verbindungen mit stärkerer Bindung zur komplementären DNA bei gleichzeitiger Erhöhung der Hydrophilie zu kommen.

Die Cooligomere eignen sich zur Kontrolle der Genexpression. Weiterhin lassen sich derartige Substanzen in Diagnostik und Molekularbiologie zur Isolierung, Identifizierung und Quantifizierung von Nukleinsäuren verwenden.

Bei der Bearbeitung derartiger Strukturen gelang die Synthese neuer Hydroxyethylglycin- und 3'-verknüpfter Nucleotid-Hydroxyethylglycin-Bausteine. Letztere eignen sich zur Herstellung bisher unbekannter streng alternierender PNA/DNA-Cooligomere.

Die Erfindung betrifft Verbindungen der allgemeinen Formel (I) 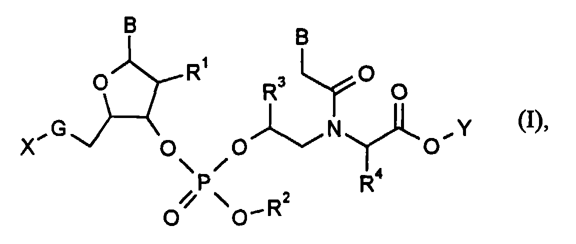 in welcher
- R¹: für Wasserstoff, Hydroxy, C₁-C₁₈-Alkoxy, Halogen, Azido oder Amino steht,
- R²: für eine in der Nucleotidchemie übliche Phosphatschutzgruppe wie beispielsweise 2-Cyanoethyl steht,
- R³ und R⁴: unabhängig voneinander für Wasserstoff, C₁-C₄-Alkyl oder gemeinsam für C₁-C₄-Alkandiyl wie beispielsweise -CH₂-, -(CH₂)₂-, -(CH₂)₃- oder -C(CH₃)₂- stehen,
- B: für alle natürlichen oder unnatürlichen Nukleobasen, wie z.B. Thymin, Uracil, Cytosin, Adenin, Guanin, Hypoxanthin oder durch chemische Modifikation von diesen abgeleitete Derivate oder halogenierte Vorstufen von diesen, gegebenenfalls an den Aminogruppen durch Schutzgruppen wie Acetyl, Trifluoracetyl, Trichloracetyl, Benzoyl, Phenylacetyl, Benzyloxycarbonyl (Z od. Cbz), tert.-Buryloxycarbonyl (Boc), Allyloxycarbonyl, (9-Fluorenyl)methoxycarbonyl (Fmoc) oder andere in der Peptid- und Nukleinsäurechemie übliche Schutzgruppen substituiert, steht,
- G: für -NH- oder -O- steht,
- X: für Wasserstoff oder eine beliebige aus der Peptidchemie bekannte Schutzgruppe wie beispielsweise Z, Fmoc oder 4-Methorytrityl steht und
- Y: für Wasserstoff, C₁-C₆-Alkyl oder eine beliebige aus der Peptidchemie bekannte C-terminale Schutzgruppe wie beispielsweise Benzyl oder tert.-Butyl steht.

Die erfindungsgemäßen Verbindungen der Formel (I) eignen sich als DNA/PNA-Bausteine zur Herstellung von DNA/PNA-Cooligomeren beispielsweise nach Standardverfahren insbesondere der Peptidchemie für Veresterungen und Amidierungen in fester und flüssiger Phase (vgl. EP 700 928 A1, S. 5 und dort zit. Lit.).

Die erfindungsgemäßen DNA/PNA-Bausteine sind durch die Formel (I) allgemein definiert.
- R¹: steht bevorzugt für Wasserstoff, Hydroxy, C₁-C₆-Alkoxy, Fluor, Chlor, Brom, Azido oder Amino.
- R²: steht bevorzugt für eine in der Nucleotidchemie übliche Phosphatschutzgruppe wie beispielsweise 2-Cyanoethyl.
- R³ und R⁴: stehen bevorzugt unabhängig voneinander für Wasserstoff, C₁-C₄-Alkyl oder gemeinsam für C₁-C₄-Alkandiyl wie beispielsweise -CH₂-, -(CH₂)₂-, -(CH₂)₃- oder -C(CH₃)₂-.
- B: steht bevorzugt für alle natürlichen Nukleobasen, wie z.B. Thymin, Uracil, Cytosin, Adenin, Guanin oder Hypoxanthin oder halogenierte Vorstufen von diesen, die gegebenenfalls an den Aminogruppen durch Schutzgruppen wie Acetyl, Trifluoracetyl, Trichloracetyl, Benzoyl, Phenylacetyl, Benzyloxycarbonyl, tert.-Butyloxycarbonyl, Allyloxycarbonyl, (9-Fluorenyl)-methoxycarbonyl oder andere in der Peptid- und Nukleinsäurechemie übliche Schutzgruppen substituiert sind.
- G: steht bevorzugt für -NH- oder -O-.
- X: steht bevorzugt für Wasserstoff oder eine beliebige aus der Peptidchemie bekannte Schutzgruppe wie beispielsweise Z, Fmoc oder 4-Methoxytrityl.
- Y: steht bevorzugt für Wasserstoff, C₁-C₄-Alkyl oder eine beliebige aus der Peptidchemie bekannte C-terminale Schutzgruppe wie beispielsweise Benzyl oder tert.-Butyl.
- R¹: steht besonders bevorzugt für Wasserstoff, Hydroxy, Methoxy, Ethoxy, Fluor, Chlor, Azido oder Amino.
- R²: steht besonders bevorzugt für 2-Cyanoethyl.
- R³ und R⁴: stehen besonders bevorzugt unabhängig voneinander für Wasserstoff, Methyl, Ethyl, Isopropyl oder gemeinsam für -CH₂-, -(CH₂)₂-, -(CH₂)₃-oder -C(CH₃)₂-.
- B: steht besonders bevorzugt für alle natürlichen Nukleobasen, wie z.B. Thymin, Uracil, Cytosin, Adenin, Guanin oder Hypoxanthin oder halogenierte Vorstufen von diesen, die gegebenenfalls an den Aminogruppen durch Schutzgruppen wie Aceryl, Trifluoraceryl, Trichloracetyl, Benzoyl, Phenylacetyl, Benzyloxycarbonyl, tert.-Buryloxycarbonyl, Allyloxycarbonyl, (9-Fluorenyl)methoxycarbonyl oder andere in der Peptid- und Nukleinsäurechemie übliche Schutzgruppen substituiert sind.
- G: steht besonders bevorzugt für -NH- oder -O-.
- X: steht besonders bevorzugt für Wasserstoff oder eine beliebige aus der Peptidchemie bekannte Schutzgruppe wie beispielsweise Z, Fmoc oder 4-Methoxytrityl.
- Y: steht besonders bevorzugt für Wasserstoff, Methyl, Ethyl, n-Propyl, Isopropyl, n-Butyl, Isobutyl, sec.-Butyl, tert.-Butyl oder eine beliebige aus der Peptidchemie bekannte C-terminale Schutzgruppe wie beispielsweise Benzyl oder tert.-Butyl.

Die Erfindung betrifft weiterhin Verbindungen der allgemeinen Formel (II) 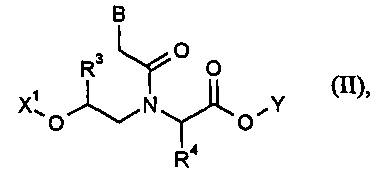 in welcher
- R³, R⁴ und B: die bei der Beschreibung der Verbindungen der Formel (I) angegebenen Bedeutungen haben,
- X¹: für Wasserstoff oder eine aus der Peptidchemie bekannte säurelabile Schutzgruppe wie beispielsweise durch Alkyl und/oder Phenyl substituiertes Silyl, Z, Fmoc oder 4-Methoxytrityl steht, wobei für den Fall, daß R³ und R⁴ gleichzeitig jeweils für Wasserstoff stehen, gegebenenfalls substituiertes Alkoxycarbonyl und Trityl ausgenommen sind und
- Y: für C₁-C₆-Alkyl oder eine beliebige aus der Peptidchemie bekannte C-terminale Schutzgruppe wie beispielsweise Benzyl oder tert.-Butyl steht.

Die erfindungsgemäßen PNA-Bausteine sind durch die Formel (II) allgemein definiert.
- R³, R⁴ und B: stehen bevorzugt für die bei der Beschreibung der Verbindungen der Formel (I) als bevorzugt angegebenen Reste.
- X¹: steht bevorzugt für Wasserstoff oder eine aus der Peptidchemie bekannte säurelabile Schutzgruppe wie beispielsweise durch Alkyl und/oder Phenyl substituiertes Silyl (z.B. tert.-Butyldimethylsilyl oder tert.-Butyldiphenylsilyl), Z, Fmoc oder 4-Methoxytrityl steht, wobei für den Fall, daß R³ und R⁴ gleichzeitig jeweils für Wasserstoff stehen, gegebenenfalls substituiertes Alkoxycarbonyl und Trityl ausgenommen sind.
- Y: steht bevorzugt für C₁-C₄-Alkyl oder eine beliebige aus der Peptidchemie bekannte C-terminale Schutzgruppe wie beispielsweise Benzyl oder tert.-Butyl.
- R³, R⁴ und B: stehen besonders bevorzugt für die bei der Beschreibung der Verbindungen der Formel (I) als besonders bevorzugt angegebenen Reste.
- X¹: steht bevorzugt für Wasserstoff oder durch Alkyl und/oder Phenyl substituiertes Silyl wie beispielsweise tert.-Butyldimethylsilyl oder tert.-Butyldiphenylsilyl.
- Y: steht besonders bevorzugt für Methyl, Ethyl, n-Propyl, Isopropyl, n-Butyl, Isobutyl, sec.-Butyl, tert.-Butyl oder eine beliebige aus der Peptidchemie bekannte C-terminale Schutzgruppe wie beispielsweise Benzyl oder tert.-Butyl.

Die aus der Peptidchemie bekannten Schutzgruppen sind z.B. aufgeführt in T. W. Greene, P. G. M. Wuts, Protective Groups in Organic Synthesis, 2^{nd} Ed., John Wiley & Sons, New York 1991.

Die oben aufgeführten allgemeinen oder in Vorzugsbereichen aufgeführten Restedefinitionen bzw. Erläuterungen können untereinander, also auch zwischen den jeweiligen Bereichen und Vorzugsbereichen beliebig kombiniert werden. Sie gelten für die Endprodukte sowie für die Vor- und Zwischenprodukte entsprechend.

Erfindungsgemäß bevorzugt werden die Verbindungen der Formel (I), in welchen eine Kombination der vorstehend als bevorzugt (vorzugsweise) aufgeführten Bedeutungen vorliegt.

Erfindungsgemäß besonders bevorzugt werden die Verbindungen der Formel (I), in welchen eine Kombination der vorstehend als besonders bevorzugt aufgeführten Bedeutungen vorliegt.

Gesättigte oder ungesättigte Kohlenwasserstoffreste wie Alkyl oder Alkenyl können, auch in Verbindung mit Heteroatomen, wie z.B. in Alkoxy, soweit möglich, jeweils geradkettig oder verzweigt sein.

Gegebenenfalls substituierte Reste können einfach oder mehrfach substituiert sein, wobei bei Mehrfachsubstitutionen die Substituenten gleich oder verschieden sein können.

Beispiele für die erfindungsgemäßen Verbindungen der Formel (I) sind Bausteine der Formel (I-a): 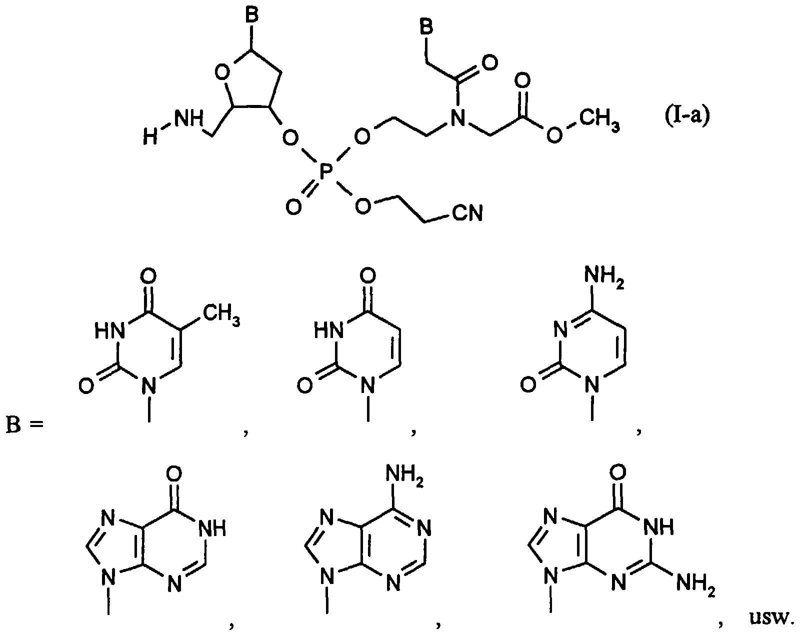
A) DNA/PNA-Bausteine der Formel (I) 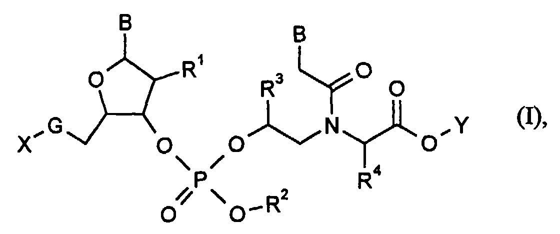 in welcher
   R¹ bis R⁴, B, G, X und Y die oben angegebene Bedeutung haben,
   lassen sich herstellen, indem man Verbindungen der Formel (II-a) 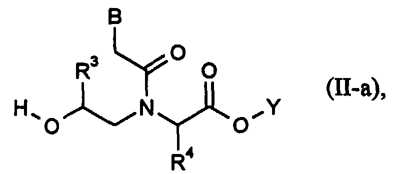 mit Nukleotid-Derivaten der Formel (III) 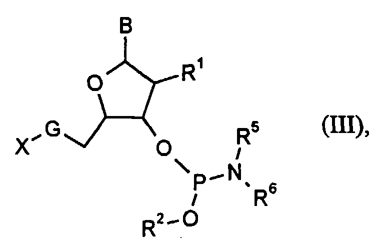 in welcher
   R¹, R², B, G und X die oben angegebene Bedeutung haben und
   R⁵ und R⁶ unabhängig jeweils voneinander für C₁-C₄-Alkyl stehen,
   in Gegenwart eines Verdünnungsmittels und eines Reaktionshilfsmittels umsetzt und anschließend oxidiert.
B) PNA-Bausteine der Formel (II) 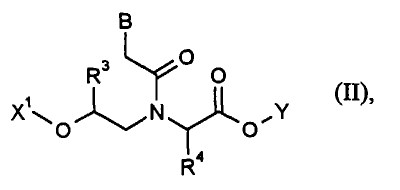 in welcher
   R³, R⁴, B, X¹ und Y die oben angegebene Bedeutung haben
   lassen sich herstellen, in dem man geschützte Hydroxyethylglycin-Derivate der Formel (IV) 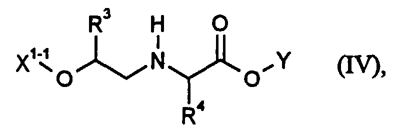 in welcher
   - X¹⁻¹: mit Ausnahme von Wasserstoff dieselbe Bedeutung wie X¹ hat,
   mit durch Nukleobasen substituierter Essigsäuren der Formel (V)

   B―CH₂-COOH (V)

   in Gegenwart eines Reaktionshilfsmittels und eines Verdünnungsmittels und gegebenenfalls in Gegenwart einer Base umsetzt und für den Fall, daß X¹ = H sein soll [Formel (II-a)], anschließend die Schutzgruppe mit einem geeigneten Reagenz abspaltet.

Verwendet man beispielsweise N-(2-Hydroxy-2-methyl-ethyl)-N-[(N⁶-benzyloxycarbonyl-adenin-1-yl)-acetyl]-glycin-isopropylester und 2-Cyanoethyl-N,N-di-isopropyl-5'-O-(Dimethoxytrityl)-thymidin-3'-phosphoramidit als Ausgangsstoffe, so kann der Reaktionsablauf des erfindungsgemäßen Verfahrens (A) durch das folgende Formelschema wiedergegeben werden:

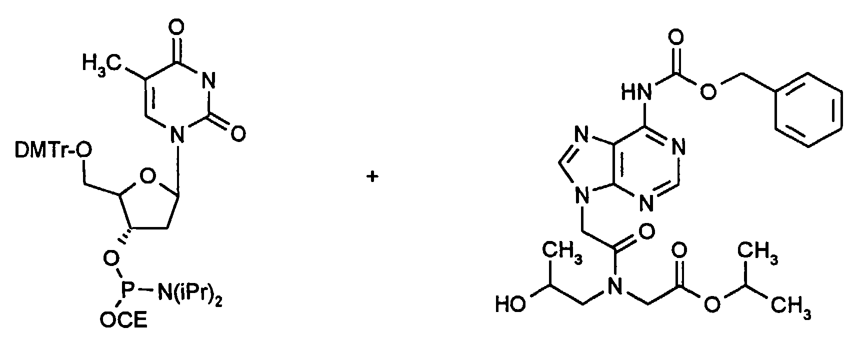

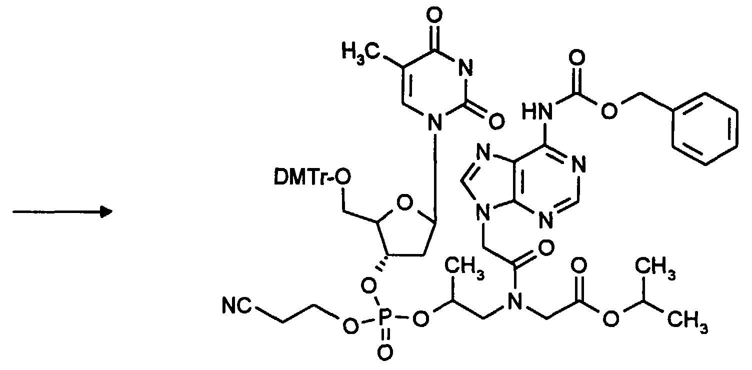

Verwendet man beispielsweise (N⁶-Benzyloxycarbonyl-adenin-1-yl)-essigsäure und [N-(2-Dimethyl-^{t}butylsilyloxy)-ethyl]-alanin-ethylester als Ausgangsstoffe, so kann der Reakionsablauf des erfindungsgemäßen Verfahrens (B) durch das folgende Formelschema wiedergegeben werden:

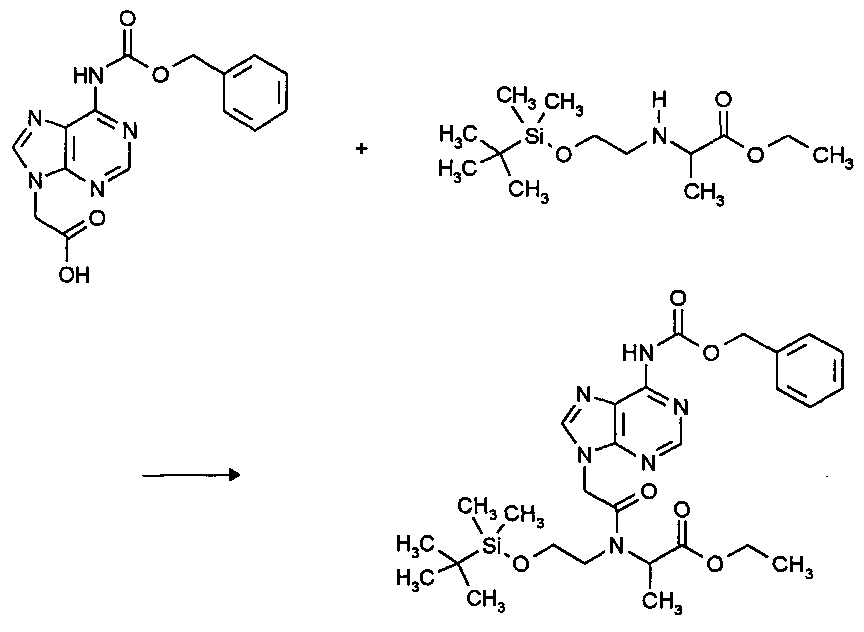

Die zur Durchführung des erfindungsgemäßen Verfahrens (A) benötigten PNA-Bausteine der Formel (II-a) sind eine Teilmenge der neuen Verbindungen der Formel (II), in der X¹ für Wasserstoff steht. Sie lassen sich z.B. nach Verfahren (B) herstellen.

Die weiterhin zur Durchfürung des erfindungsgemäßen Verfahrens (A) benötigten Nukleotid-Derivate sind durch die Formel (III) allgemein definiert. In dieser Formel stehen R¹, R², G und X vorzugsweise für diejenigen Reste, die bereits bei der Beschreibung der Bausteine der Formel (I) als bevorzugt genannt wurden. R⁵ und R⁶ stehen bevorzugt jeweils für Methyl, Ethyl, n-, sec.- oder iso-Propyl.

Die Verbindungen der Formel (III) sind bekannt oder lassen sich nach bekannten Methoden herstellen (vgl. M. J. Gait (ed.) Oligonucleotide Synthesis, A Practical Approach, IRL Press, Oxford 1984).

Die zur Durchführung des erfindungsgemäßen Verfahrens (B) benötigten Hydroxyethylglycin-Derivate sind durch die Formel (IV) allgemein definiert. In dieser Formel stehen R³, R⁴ und Y vorzugsweise für diejenigen Reste, die bereits bei der Beschreibung der Bausteine der Formel (I) als bevorzugt genannt wurden. X¹⁻¹ steht vorzugsweise, Wasserstoff ausgenommen, für dieselben Reste, die vorzugsweise für X¹ in Formel (II) stehen.

Verbindungen der Formel (IV) lassen sich z.B. herstellen, indem man die Schutzgruppe X² von vollständig geschützter Hydroxyethylglycin-Derivate der Formel (VI) gemäß dem folgenden Realttionsschema abspaltet:

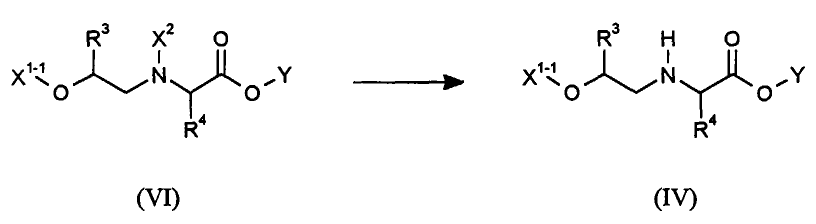

In der Formel (VI) steht X² für eine in Abhängigkeit von Y geeignete N-terminale Schutzgruppe wie beispielsweise tert.-Butoxycarbonyl (BOC), Benzyloxycarbonyl (Cbz) oder Benzyl (Bzl), bevorzugt für Bzl oder Cbz (Vgl. z.B. T. W. Greene, P. G. M. Wuts, Protective Groups in Organic Synthesis, 2. Ed., John Wiley & Sons, New York 1991).

Die Reaktion läßt sich mittels üblicher Methoden N-terminaler Deblockierung wie Acidolyse, beispielsweise im Falle der BOC-Gruppe, oder katalytischer Hydrierung, beispielsweise im Falle eines Benzylesters, durchführen.

Verbindungen der Formel (VI) lassen sich z.B. herstellen, indem man die Hydroxygruppe der Hydroxyethylglycin-Derivate der Formel (VII) gemäß folgendem Reaktionsschema nach üblichen Methoden schützt (vgl. o.a. Lit.):

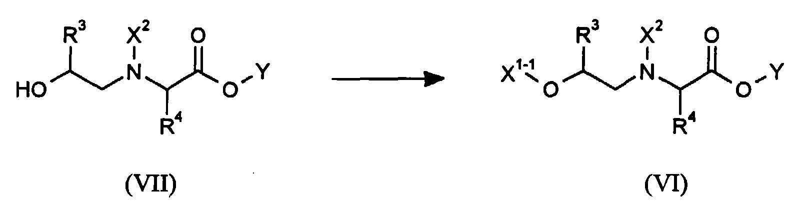

Verbindungen der Formel (VII) lassen sich z.B. herstellen, indem man die Carboxylgruppe der Hydroxyethylglycin-Derivate der Formel (VIII) gemäß folgendem Reaktionsschema nach üblichen Methoden verestert oder schützt:

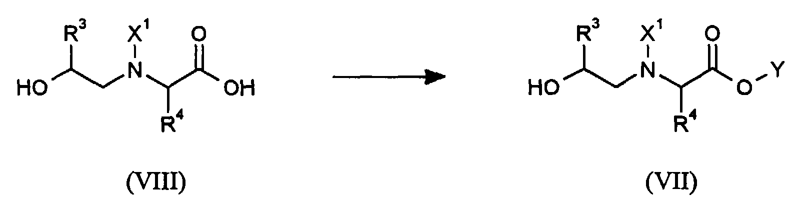

Hydroxyethylglycin-Derivate der Formel (VIII) sind zum Teil kommerziell erhältlich, bekannt oder lassen sich nach bekannten Methoden herstellen.

Spezielle Verbindungen (VII-a) der Formel (VII) lassen sich z.B. auch herstellen, indem man N-geschützte Hydroxyethanole der Formel (IX) mit α-Bromcarbonsäurederivaten der Formel (X) nach üblichen Methoden gemäß folgendem Reaktionsschema umsetzt:

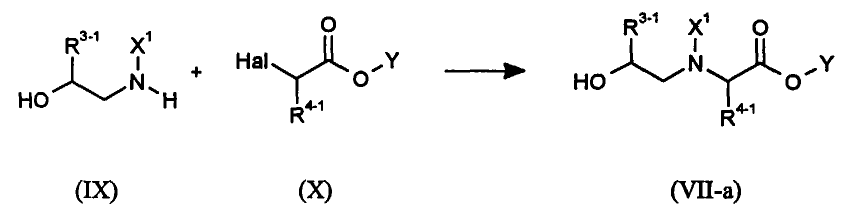

In den Formeln (VII-a), (IX) und (X) stehen R³⁻¹ und R⁴⁻¹ unabhängig voneinander für Wasserstoff oder C₁-C₄-Alkyl.

Verbindungen der Formel (IX) und (X) sind kommerziell erhältlich, allgemein bekannt oder lassen sich nach bekannten Methoden herstellen (vgl. Burfiled et al., J. Chem. Soc. Perkin Trans. I, **1977**, 666).

Die weiterhin zur Durchführung des erfindungsgemäßen Verfahrens (B) benötigten substituierten Essigsäuren der Formel (V) sind bekannt oder lassen sich nach bekannten Methoden herstellen (vgl. Dueholm et al. J Org. Chem. **59**, 5767 (1994)).

Das erfindungsgemäße Verfahren (A) wird bevorzugt in Lösung durchgeführt, kann aber auch an fester Phase erfolgen (siehe: R. B. Merrifield, J. Am. Chem. Soc. **85**, 2149 (1963) und M. J. Galt, (ed.) Oligonucleotide Synthesis, A Practical Approach, IRL Press, Oxford 1984).

Als Reaktionshilfsmittel zur Durchführung der Phophotriesterbildung des erfindungsgemäßen Verfahrens (A) eignen sich alle Aktivatoren, die zur Bildung einer Phosphitbindung geeignet sind und alle Oxidationsmittel, die zur Überführung des Phosphits in das entsprechende Phosphat geeignet sind (Shabarova, Z.; Bogdanov, A., Advanced Organic Chemistry of Nucleic Acids, Verlag Chemie, Weinheim 1994). Vorzugsweise kommen folgen Aktivatoren in Frage: Azole wie Tetrazole, Triazole sowie deren Ammoniumsalze. Als Oxidationsmittel kommen bevorzugt in Frage: Persäuren wie 3-Chlorperbenzoe-säure, Lösungen von Iod in Lösemittelgemischen aus Wasser, THF, tert. Aminen.

Als Verdünnungsmittel zur Durchführung des erfindungsgemäßen Verfahrens (A) kommen organische Lösungsmittel und beliebige Mischungen davon in Betracht. Beispielhaft seien genannt: aliphatische, alicyclische oder aromatische Kohlenwasserstoffe, wie beispielsweise Petrolether, Hexan, Heptan, Cyclohexan, Methylcyclohexan, Benzol, Toluol, Xylol oder Decalin; halogenierte Kohlenwasserstoffe, wie beispielsweise Chlorbenzol, Dichlorbenzol, Methylenchlorid, Chloroform, Tetrachlormethan, Dichlor-, Trichlorethan oder Tetrachlorethylen; Ether, wie Diethyl-, Diisopropyl-, Methyl-t-butyl-, Methyl-t-amylether, Dioxan, Tetrahydrofuran, 1,2-Dimethoxyethan, 1,2-Diethoxyethan, Diethylenglykoldimethylether oder Anisol; Ketone, wie Aceton, Butanon, Methyl-isoburylketon oder Cyclohexanon; Nitrile, wie Acetonitril, Propionitril, n- oder i-Butyronitril oder Benzonitril; Amide, wie Formamid, N,N-Dimethylformamid, N,N-Dimethylacetamid, N-Methylformanilid, N-Methylpyrrolidon oder Hexamethylphosphorsäuretriamid; N-Oxide wie N-Methylmorpholin-N-oxid; Ester wie Methyl-, Ethyl- oder Butylacetat; Sulfoxide, wie Dimethylsulfoxid; Sulfone, wie Sulfolan.

Die Reaktionstemperatur kann bei dem erfindungsgemäßen Verfahren (A) innerhalb eines großen Bereichs variiert werden. Im allgemeinen arbeitet man bei der Kondensation bei Temperaturen zwischen -30°C und +80°C, bevorzugt bei -10°C bis 60°C, besonders bevorzugt bei 0°C bis Raumtemperatur.

Bei der Durchführung des erfindungsgemäßen Verfahrens (A) werden pro Mol Verbindung der Formel (III) 0,5 bis 3 Mol, vorzugsweise 0,8 bis 1,5 Mol Verbindung der Formel (II) und 1 bis 3 Mol Reaktionshilfsmittel und das Oxidationsmittel im allgemeinen im molaren Verhältnis von 1:1 bis 1:4, vorzugsweise 1:2 bis 1:3, eingesetzt.

Als Reaktionshilfsmittel zur Durchführung der Amidierung des erfindungsgemäßen Verfahrens (B) eignen sich alle Verbindungen, die zur Knüpfung einer Amidbindung geeignet sind (vgl. z.B. Houben-Weyl, Methoden der Organischen Chemie, Band 15/2; Bodensky et al.; Peptide Synthesis 2nd ed., Wiley & Sons, New York 1976). Vorzugsweise kommen folgende Methoden in Frage: Aktivestermethode mit Pentafluorphenol (PfP), N-Hydroxysuccinimid, 1-Hydroxybenzotriazol (HOBt), Kopplung mit Carbodiimiden wie Dicyclohexylcarbodiimid oder N'-(3-Dimethylaminopropyl)-N-ethylcarbodiimid (EDC od. EDCI)) sowie die Gemiscbte-Anhydrid-Methode oder die Kopplung mit Phosphoniumreagenzien wie 1-Benzotriazolyloxy-tris-(dimethylaminophosphonium)-hexafluophosphat (BOP), Bis-(2-oxo-3-oxazolidinyl)-phosphorylchlorid (BOP-Cl) oder mit Phosphonsäureesterreagenzien wie Cyanphosphonsäurediethylester (DEPC) und Diphenylphosphorylazid (DPPA). Besonders bevorzugt ist die Kopplung mit BOP-Cl oder EDCI in Gegenwart von HOBt.

Als Verdünnungsmittel zur Durchführung des erfindungsgemäßen Verfahrens (B) kommen organische Lösungsmittel und beliebige Mischungen davon in Betracht. Beispielhaft seien genannt: aliphatische, alicyclische oder aromatische Kohlenwasserstoffe, wie beispielsweise Petrolether, Hexan, Heptan, Cyclohexan, Methylcyclohexan, Benzol, Toluol, Xylol oder Decalin; halogenierte Kohlenwasserstoffe, wie beispielsweise Chlorbenzol, Dichlorbenzol, Methylenchlorid, Chloroform, Tetrachlormethan, Dichlor-, Trichlorethan oder Tetrachlorethylen; Ether, wie Diethyl-, Diisopropyl-, Methyl-t-butyl-, Methyl-t-Amylether, Dioxan, Tetrahydrofuran, 1,2-Dimethoxyethan, 1,2-Diethoxyethan, Diethylenglykoldimethylether oder Anisol; Ketone, wie Aceton, Butanon, Methyl-isobutylketon oder Cyclohexanon; Nitrile, wie Acetonitril, Propionitril, n- oder i-Butyronitril oder Benzonitril; Amide, wie Formamid, N,N-Dimethylformamid, N,N-Dimethylacetamid, N-Methylformanilid, N-Methylpyrrolidon, 1,3-Dimethyl-tetrahydro-2-pyrimidinon (DMPU), 1,3-Dimethyl-2-imidazolidinon, Tetramethylharnstoff oder Hexamethylphosphorsäuretriamid; N-Oxide wie N-Methylmorpholin-N-oxid; Ester wie Methyl-, Ethyl- oder Butylacetat; Sulfoxide, wie Dimethylsulfoxid; Sulfone, wie Sulfolan.

Die Amidierung wird vorzugsweise in Gegenwart einer Base durchgeführt. Als solche kommen anorganische oder organische Basen in Frage. Hierzu gehören vorzugsweise Erdalkalimetall- oder Alkalimetallhydroxide, -alkoholate, -acetate, -carbonate oder -hydrogencarbonate, wie beispielsweise Natrium-, Kalium- oder Ammoniumhydroxid, Natrium-methylat, Natrium-ethylat, Kalium-tert.-burylat, Natrium-, Kalium-, Calcium- oder Ammoniumacetat, Natrium-, Kalium- oder Ammoniumcarbonat, Natriumhydrogen- oder Kaliumhydrogencarbonat sowie tertiäre Amine, wie Trimethylamin, Triethylamin, Tributylamin, Ethyl-diisopropylamin, N,N-Dimethylanilin, N,N-Dimethyl-benzylamin, Pyridin, Picolin, N-Methylpiperidin, N-Methylmorpholin, N,N-Dimethylaminopyridin, Diazabicyclooctan (DABCO), Diazabicyclononen (DBN) oder Diazabicycloundecen (DBU).

Die Reaktionstemperatur kann bei dem erfindungsgemäßen Verfahren (B) innerhalb eines größeren Bereiches variiert werden. Im allgemeinen arbeitet man bei der Kondensation bei Temperaturen zwischen -80°C und +150°C, bevorzugt bei -50°C bis 100°C, besonders bevorzugt bei -30°C bis Raumtemperatur.

Bei der Durchführung des erfindungsgemäßen Verfahrens (B) werden pro Mol Verbindung der Formel (IV) 1 bis 5 Mol, vorzugsweise 1,5 bis 3 Mol Essigsäure der Formel (V) und 1 bis 3 Mol Reaktionshilfsmittel und die Base im allgemeinen im molaren Verhältnis von 1:1 bis 1:3, vorzugsweise 1:1,5 bis 1:2,5, eingesetzt.

Die Abspaltung der Schutzgruppe kann nach bekannten Methoden erfolgen (Vgl. z.B. T. W. Greene, P. G. M. Wuts, Protective Groups in Organic Synthesis, 2. Ed., John Wiley & Sons, New York 1991).

Die Umsetzungen der erfindungsgemäßen Verfahren können bei Normaldruck oder unter erhöhtem Druck durchgeführt werden, vorzugsweise wird bei Normaldruck gearbeitet. Die Aufarbeitung geschieht nach üblichen Methoden der Organischen Chemie. Die Endprodukte werden vorzugsweise durch Kristallisation, chromatographische Reinigung oder durch Entfernung der flüchtigen Bestandteile gegebenenfalls im Vakuum gereinigt.

### Experimenteller Teil

### Beispiel 1: N-Benzyloxycarbonyl-4-hydroxy-L-trans-prolinmethylester

25,1 g N-Benzyloxycarbonyl-4-hydroxy-L-trans-prolin werden in 450 ml wasserfreiem Methanol gelöst und mit Cesiumcarbonat auf pH = 9,0 - 9,5 eingestellt. Nach 30minütigem Rühren bei Raumtemperatur wird bis zur Trockene eingedampft und der Rückstand über Nacht im Hochvakuum getrocknet. Der Rückstand wird in N,N-Dimethylformamid (DMF) aufgenommen, zu dieser Suspension langsam 14,8 g Methyliodid so zugetropft, daß die Temperatur der Reaktionsmischung 30°C nicht übersteigt und für weiter 12 Stunden verrührt. Das Lösemittel wird entfernt, der verbleibende Rückstand mehrfach mit Toluol coevaporiert und in Dichlormethan aufgenommen. Man wäscht die Lösung mit Wasser/ges. Kochsalzlösung 1:1 (v/v), 5%iger NaHCO₃-Lsg. und erneut Wasser/Kochsalzlösung. Die organische Phase wird über MgSO₄ getrocknet, eingeengt und das resultierende Öl im Hochvakuum getrocknet.
- Ausbeute:: 21,86 g
- R_{f}:: 0,15 Laufmittel: Toluol / Ethanol 10:1

### Beispiel 2: N-Z-4-(^{t}Butyldimethylsilyloxy)-L-trans-prolinmethylester

Unter Argon werden 14,4 g (51,7 mmol) N-Z-4-hydroxy-L-trans-prolinmethylester in 150 mi N, N-Dimethylformamid gelöst und mit 14,8 ml Triethylamin versetzt. Innerhalb zehn Minuten werden 10,4 g (68,8 mmol) ^{t}Butyldimethylsilylchlorid (TBDMSi-Chlorid) zugetropft und über Nacht nachgerührt. Nach Entfernen des Lösungsmittels im Vakuum verbleibt ein Öl, das zwischen Dichlormethan und Wasser partitioniert wird. Man extrahiert die wäßrige Phase noch mehrmals mit Dichlormethan, trocknet über Natriumsulfat und verdampft das Lösemittel im Vakuum. Anschließend wird durch Chromatographie an Kieselgel mit Dichlormethan/Methanol (60:1, v/v) als Eluens gereinigt.
- Ausbeute:: 13,95 g
- R_{f}:: 0,54 Laufmittel: CH₂Cl₂ / MeOH 40:1

### Beispiel 3: 4-(^{t}Butyldimethylsilyloxy)-L-trans-prolinmethylester

Die Verbindung aus Beispiel 2 (3,8 g; 9,6 mmol) wird in wasserfreiem Methanol (40 ml) gelöst und bei Normaldruck und Raumtemperatur über Palladium/Aktivkohle (10%; 0,3 g) vier Stunden hydriert. Nach vollständiger Umsetzung wird der Katalysator abfiltriert und in Vakuum eingeengt.
- Ausbeute:: 2,4 g
- R_{f}:: 0,52 Laufmittel: CH₂Cl₂ / MeOH 10:1

### Beispiel 4: 4-(^{t}Butyldimethylsilyloxy)-N-[(thymin-1-yl)-acetyl]-L-trans-prolinmethylester

3,4 g (18,5 mmol) 1-Carboxymethyl-thymin werden in 50 ml DMF gelöst, auf - 30°C abgekühlt und bei dieser Temperatur mit 1,3 eq. HOBt*H₂O und 1,3 eq. EDCI*HCl versetzt. Man rührt 30 min bei -30°C und gibt eine Lösung von 2,4 g (19,3 mmol) 4-(^{t}Butyldimethylsilyloxy)-L-trans-prolinmethylester in DMF und 4,5 ml Triethylamin zu. Eine Stunde wird bei -30°C verrührt und anschließend noch über Nacht bei Raumtemperatur nachgerührt. Dann wird eingeengt, der Rückstand in Essigsäureethylester aufgenommen und jeweils mit 1 N HCl, gesättigter

Natriumhydrogencarbonatlösung und gesättigter NaCl-Lösung gewaschen. Das Produkt knstaltisiert über Nacht aus der organischen Phase aus, wird abfiltriert und im Hochvakuum getrocknet.
- Ausbeute:: 3,03 g
- R_{f}:: 0,68 Laufmittel: CH₂Cl₂ / MeOH 9:1

### Beispiel 5: 4-Hydroxy- N-[(thymin-1-yl)-acetyl]-L-trans-prolinmethylester

2,0 g 4-(^{t}Butyldimethylsilyloxy)-N-[(thymin-1-yl)-acetyl]-L-trans-prolinmethylester werden in 50 ml Methanol gelöst, mit 5 Tropfen konzentrierter Salzsäure versetzt und bei Raumtemperatur über Nacht gerührt. Die Lösung wird im Hochvakuum vollständig eingedampft und der Rückstand durch Chromatographie an Kieselgel mit Dichlormethan/Methanol (60:1, v/v) als Eluens gereinigt.
- Ausbeute:: 1,19 g
- R_{f}:: 0,21 Laufmittel: CH₂Cl₂ / MeOH 20:1

### Beispiel 6: N-Benzyl-N-(2-hydroxyethyl)-glycin-^{t}butylester

Eine Lösung von 9,6 g (63,4 mmol) N-Benzylethanolamin und 8,85 ml Triethylamin in 65 ml N, N-Dimethylformamid wird bei 0°C mit 1 eq. Bromessigsäure-^{t}butylester versetzt und noch 22 h bei Raumtemperatur nachgerührt. Nach Einengen wird mehrmals mit Toluol co-destilliert, der Rückstand in Dichlormethan aufgenommen und mehrfach mit Wasser extrahiert. Die organische Phase wird zur Trockene eingedampft, das verbleibende Öl im Hochvakuum getrocknet.
- Ausbeute:: 14,5 g
- R_{f}:: 0,47 Laufmittel: EE (Essigsäureethylester)

### Beispiel 7: N-Benzyl-N-(2-hydroxyethyl)-glgcinmethylester

Die zu Beispiel 6 analoge Umsetzung von 41,9 g (277 mmol) N-Benzylethanolamin mit Bromessigsäure-methylester liefert N-Benzyl-N-(2-hydroxyethyl)-glycinmethylester.
- Ausbeute:: 43,8 g
- R_{f}:: 0,53 Laufmittel: CH₂Cl₂ / MeOH 20:1

### Beispiel 8: N-Benzyl-N-(2-hydroxyethyl)-glycinmethylester

Die zu Beispiel 6 analoge Umsetzung von 34,4 g (227 mmol) N-Benzylethanolamin mit Bromessigsäure-ethylester liefert N-Benzyl-N-(2-hydroxyethyl)-glycinethylester.
- Ausbeute:: 43,9 g
- R_{f}:: 0,49 Laufmittel: CH₂Cl₂ / MeOH 20:1

### Beispiel 9: N-Benzyl-N-(2-^{t}butyldiphenylsilyloxyethyl)-glycin-^{t}butylester

Unter Argon werden 5,2 g (19,5 mmol) N-Benzyl-N-(2-hydroxyethyl)-glycin-^{t}butylester in 50 mi DMF gelöst und mit 5,5 ml Triethylamin versetzt. Es wird eine Lösung von 1,3 eq TBDPSi-Chlorid zugetropft und über Nacht nachgerührt. Das Lösungsmittel wird entfernt, der Rückstand zwischen Wasser und Dichlormethan verteilt. Die wäßrige Phase wird noch dreimal mit Dichlormethan extrahiert und die vereinigten organischen Phasen anschließend über Natriumsulfat getrockhet. Das Rohprodukt wird durch Chromatographie an Kieselgel mit Essigsäureethylester/Hexan (1:9, v/v) als Eluens gereinigt.
- Ausbeute:: 6,79 g
- R_{f}:: 0,68 Laufmittel: EE / Hexan 1:9

### Beispiel 10: N-Benzyl-N-(2-^{t}butyldimethylsiyloxy-ethyl)-glycinmethylester

Die zu Beispiel 9 analoge Umsetzung von 37,7 g (170 mmol) N-Benzyl-N-(2-hydroxyethyl)-glycinmethylester mit ^{t}Butyldimethylsilylchlorid liefert N-Benzyl-N-(2-^{t}butyldimethylsilyloxy-ethyl)glycinmethylester nach Hochvakuumtrocknung als Öl.
- Ausbeute:: 45,1 g
- R_{f}:: 0,39 Laufmittel: EE / Hexan 1:9

### Beispiel 11: N-Benzyl-N-(2-^{t}buryldimethylsilyloxyethyl)-glycinethylester

30,1 g (127 mmol) N-Benzyl-N-(2-^{t}butyldimethyl-silyloxyethyl)-glycinethylester werden unter den Bedingungen aus Beispiel 10 mit 1,3 eq. TBDMSi-Chlorid umgesetzt.
- Ausbeute:: 27,3 g
- R_{f}:: 0,49 Laufmittel: EE / Hexan 1:10

### Beispiel 12: N-(2-^{t}Butyldiphenylsilyloxyethyl)-glycin-^{t}butylester

Zur Entfernung der Benzylschutzgruppe werden 5,9 g (11,7 mmol) N-Benzyl-N-(2-^{t}butyldiphenylsilyloxy-ethyl)-glycin-^{t}butylester in 50 ml wasserfreiem Methanol gelöst und bei Raumtemperatur und Normaldruck über Palladium/Aktivkohle (10%; 0,5 g) für 36 Stunden hydriert. Nach vollständiger Umsetzung (DC-Kontrolle) wird der Katalysator abfiltriert und im Vakuum eingedampft.
- Ausbeute:: 4,91 g
- R_{f}:: 0,46 Laufmittel: EE / PE (Petrolether) 1:9

### Beispiel 13: N-(2-^{t}Butyldimethylsilyloxy-ethyl)-glycinmethylester

Die analoge Hydrierung von 23,8 g (71 mmol) der Verbindung aus Beispiel 10 liefert N-(2-^{t}butyldimethylsilyloxy-ethyl)-glycinmethylester.
- Ausbeute:: 16,7 g
- R_{f}:: 0,24 Laufmittel: EE / PE 1:1

### Beispiel 14: N-(2-^{t}Butyldimethyl-silyloxyethyl)-glycinethylester

Die zu Beispiel 13 analoge Umsetzung von 20,0 g (57,0 mmol) N-Benzyl-N-(2-^{t}butyldimethylsilyloxyethyl)-glycinethylester liefert den gewünschten Glycinethylester als Öl.
- Ausbeute:: 13,5 g
- R_{f}:: 0,61 Laufmittel: CH₂Cl₂ / MeOH 20:1

### Beispiel 15: N-(2-^{t}Butyldiphenylsilyloxy-ethyl)-N-(thymin-1-yl-aceryl)-glycin-^{t}butylester

4,4 g (23,6 mmol) 1-Carboxymethyl-thymin werden in 50 ml DMF gelöst, auf -30°C abgekühlt und bei dieser Temperatur mit HOBt*H₂O und EDCI*HCl (jeweils 1,3 eq.) versetzt. Nach 30 min wird bei dieser Temperatur eine Lösung von 4,9 g (11,8 mmol) der Verbindung aus Beispiel 12 und 6 ml Triethylamin zu gegeben. Es wird noch eine weitere Stunde bei -30°C und anschließend 12 h bei Raumtemperatur gerührt. Dann wird eingeengt, der Rückstand in Essigsäureethylester aufgenommen und jeweils mit 1 N HCl, ges. NaHCO₃-Lsg. und ges. NaCl-Lsg. gewaschen. Das Rohprodukt wird durch Chromatographie an Kieselgel mit Dichlormethan/Methanol (10:1, v/v) als Eluens gereinigt und fällt nach Trocknen als weißer Schaum an.
- Ausbeute:: 3,3 g
- R_{f}:: 0,73 Laufmittel: CH₂Cl₂ / MeOH 9:1

### Beispiel 16: N-(2-^{t}Butyldimethylsilylozy-ethyl)-N-(thymin-1-yl-acetyl)-glycinmethylester

Die zu Beispiel 15 vergleichbare Umsetzung mit 10,0 g (40,4 mmol) der Substenz aus Beispiel 13 liefert N-(2-^{t}Butyldimethylsilyloxyethyl)-N-(thymin-1-yl-acetyl)-glycinmethylester als weißen Feststoff.
- Ausbeute:: 3,55g
- R_{f}:: 0,60 Laufmittel: CH₂Cl₂ / MeOH 10:1

### Beispiel 17: N-(2-^{t}Butyldimethylsilylosy-ethyl)-N-(thymin-1-yl-acetyl)-glycinethylester

In einer zu Beispiel 16 analogen Reaktion werden 3,55 g (13,6 mmol) der Verbindung aus Beispiel 14 eingesetzt.
- Ausbeute:: 3,13 g
- R_{f}:: 0,63 Laufmittel: CH₂Cl₂ / MeOH 5:1

### Beispiel 18: N-(2-^{t}Butyldimethylsilyloxy-ethyl)-N-[(N⁴-Z-cytosin-1-yl)-acetyl]-glycinethylester

Die zu Beispiel 15 vergleichbare Umsetzung von 0,86 g (3,3 mmol) N-(2-^{t}butyldimethlsiyloxy-ethyl)-glycinethylester mit 2,0 g (6,6 mmol) (N⁴-Z-cytosin-1-yl)-essigsäure liefert N-(2-^{t}Butyldimethylsilyloxy-ethyl)-N-[(N⁴-Z-cytosin-1-yl)-acetyl]-glycinethylester als weißen Feststoff.
- Ausbeute:: 1,08 g
- R_{f}:: 0,61 Laufmittel: CH₂Cl₂ / MeOH 20:1

### Beispiel 19: N-(2-Hydroxyethyl)-N-(thymin-1-yl-aceryl)-glycin-^{t}butylester

Der silylgeschützte Verbindung aus Beispiel 15 (3,2 g; 5,5 mmol) wird bei 0°C in 20 ml THF gelöst und mit 3 eq. Tetrabutylammoniumfluorid (TBAF) in Form einer 1,1 M Lösung in THF versetzt. Man läßt auf Raumtemperatur kommen und rührt noch 3 Stunden nach (DC-Kontrolle). Das Lösemittel wird entfernt, der Rückstand in Essigsäureethylester aufgenommen und mit ges. NaHCO₃-Lsg sowie ges. NaCl-Lösung gewaschen. Nach Trocknen über Natriumsulfat wird das Rohprodukt durch Chromatagraphie an Kieselgel mit Dichlormethan/Methanol (9:1, v/v) als Eluens gereinigt.
- Ausbeute:: 1,10 g
- R_{f}:: 0,41 Laufmittel: CH₂Cl₂ / MeOH 10:1

### Beispiel 20: N-(2-Hydroxyethyl)-N-(thymin-1-yl-acetyl)-glycinmethylester

4,0 g (9,7 mmol) des silylgeschützten Alkohols aus Beispiel 16 werden in 30 ml Methanol suspendiert und mit konz. HCl auf pH = 1 gebracht. Nach 3 Stunden wird das Produkt abfiltriert, mit Methanol gewaschen und getrocknet.
- Ausbeute:: 1,63 g
- R_{f}:: 0,38 Laufmittel: CH₂Cl₂ / MeOH 10:1

### Beispiel 21: N-(2-Hydroxyethyl)-N-(thymin-1-yl-acetyl)-glycinethylester

Zur Desilylierung setzt man 2,2 g (5,2 mmol) der Substanz aus Beispiel 17 in 60 ml Ethanol mit 2-3 Tropfen konz. HCl um und läßt die Lösung über Nacht bei Raumtemperatur nachrühren. Das Reaktionsprodukt wird abfiltriert, nachgewaschen und im Hochvakuum getrocknet.
- Ausbeute:: 1,01 g
- R_{f}:: 0,29 Laufmittel: CH₂Cl₂ / MeOH 20:1

### Beispiel 22: N-(2-Hydroxyethyl)-N-[(N⁴-Z-cytosin-1-yl)-acetyl]-glyeinethylester

Zu einer Lösung von 0,4 g (2,7 mmol) Cesiumfluorid in 20 ml Acetonitril werden portionsweise 0,5 g (0,9 mmol) der Verbindung aus Beispiel 18 hinzugegeben.

Man läßt 24 h bei Raumtemperatur rühren und gibt dann dieselbe Menge Cesiumfluorid nochmals hinzu. Nach weiteren 24 h Rühren bei 25°C wird filtriert, das Filtrat eingedampft und durch Chromatographie an Kieselgel mit Dichlormethan/Methanol (80:1, v/v) als Eluens weiter aufgereinigt.
- Ausbeute:: 69 mg
- R_{f}:: 0,28 Laufmittel: CH₂Cl₂ / MeOH 20:1

### Beispiel 23: 5'-Azido-5'-deoxythymidin

5,0 g (20 mmol) im Hochvakuum zwei Tage über Phosphorpentoxid getrocknetes Thymidin werden in 100 ml DMF unter Argon verrührt und mit 1,25 eq Triphenylphosphin, 1,25 eq Tetrabromkohlenstoff und 3 eq. Lithiumazid versetzt. Die gelbe Lösung wird über Nacht bei Raumtemperatur gerührt. Nach Zugabe von 30 ml Methanol wird noch 90 Minuten nachgerührt und anschließend zur Trockene eingedampft. Das erhaltene Rohprodukt wird schließlich an Kieselgel mit Dichlormethan/Methanol (40:1, v/v) als Eluens chromatographiert
- Ausbeute:: 3,72
- R_{f}:: 0,32 Laufmittel: CH₂Cl₂ / MeOH 20:1

### Beispiel 24: 5'-Amino-5'-deoxythymidin

4,05 g (15,2 mmol) 5'-Azido-5'-deoxythymidin werden in Pyridin gelöst und mit 1,5 eq. Triphenylphosphin bei Raumtemperatur für drei Stunden gerührt. Anschließend werden 20 eq. Wasser zugegeben und über Nacht weitergerührt. Nach Zugabe von 120 ml Wasser wird die erhaltene Suspension abgesaugt. Das Filtrat wird fünfmal mit Essigsäureethylester gewaschen und zur Trockne eingedampft. Das zurückbleibende Pulver wird mehrmals mit Pyridin/Toluol coevaporiert und im Hochvakuum getrocknet.
- Ausbeute:: 2,78 g
- R_{f}:: 0,43 Laufmittel: CH₂Cl₂ / MeOH 10:1

### Beispiel 25: 5'-N-(4-Methozytrityl)-5'-amino-5'-deoxythymidin

2,7 g (11,5 mmol) 5'-Amino-5'-deoxythymidin werden in Pyridin vorgelegt und mit 3 eq. 4-Methoxytritylchlorid, 1 eq. Triethylamin sowie einer katalytischen Menge DMAP versetzt. Über Nacht wird unter Lichtausschluß gerührt und anschließend mit Methanol gequencht. Das Lösungsmittel wird abdestilliert, der Rückstand in Dichlormethan aufgenommen und mit 5%iger NaHCO₃-Lsg. (2x) sowie ges. NaCl-Lsg. gewaschen. Die organsche Phase wird über Natriumsulfat getrocknet und dann zur Trockene eingedampft. Das verbleibende Öl wird an basichem Aluminiumoxid mit Dichlormethan/Methanol (100:0, 100:2, 100:4, 100:6, 100:8, 100:10 (v/v) mit jeweils 1% Ammoniak) chromatographiert. Das so erhaltene Produkt wird in Dichlormethan/Triethylamin 10:1 (v/v) gelöst und durch Zugabe zu 800 ml Pentan bei 0°C gefällt. Nach 30 min bei dieser Temperatur wird der Niederschlag abfiltriert und im Hochvakuum getrocknet.
- Ausbeute:: 2,77 g
- R_{f}:: 0,46 Laufmittel: CH₂Cl₂ / MeOH 10:1

### Beispiel 26: 5'-N-(9-Fluorenylmethyloxycarbonyl)-5'-amino-5'-deoxythymidin

3,2 g (13,3 mmol) 5'-Amino-5'-deoxythymidin werden in DMF vorgelegt, mit 1,2 eq. Diisopropylethylamin sowie 1,2 eq. 9-Fluorenylmethyl-succinimidylcarbonat versetzt und bei Raumtemperatur 30 Minuten verrührt. Nach beendeter Reaktion werden 175 ml ges. NaHCO₃-Lsg. zugegeben. Der entstandene Niederschlag wird abgetrennt und gründlich mit Wasser gewaschen. Anschließend löst man in Dichlormethan und chromatographiert an basichem Aluminiumoxid mit Dichlormeth/Methanol (100:0, 100:1, 100:2 .... 100:10 (v/v) mit jeweils 1% Ammoniak).
- Ausbeute:: 1,56 g
- R_{f}:: 0,40 Laufmittel: CH₂Cl₂ / MeOH 30:1

### Beispiel 27: [N-(Ethoxycarboxymethyl)-N-(thymin-1-yl-acetyl)-2-aminoethyl]-2-cyanoethyl-5'-O-(dimethoxytrityl)-thymidin-3'-phosphat

Sowohl 0,2 g (0,64 mmol) der Substanz aus Beispiel 21 als auch 0,37 g (0,5 mmol) 2-Cyanoethyl-N,N-diisopropyl-5'-O-(Dimethoxytrityl)-thymidin-3'-phosphoramidit werden durch mehrmaliges Co-evaporieren mit Dichlormethan und anschließende getrennte Hochvakuumtrocknung für 12 h von den letzten Feuchtigkeitsspuren befreit. Unter Argon löst man die Verbindung aus Beispiel 21 in 10 ml Dichlormethan/Acetonitril (v/v) 1:1 und gibt das Phosphoramidit gefolgt von 37,5 mg (0,5 mol) Tetrazol, beide ebenfalls in diesen Lösemittelgemisch gelöst, mit einer Spritze zu. Nach beendeter Reaktion (DC-Kontrolle, ca. 5 Stunden) werden 1,2 mmol m-CPBA zugesetzt und über Nacht bei Raumtemperatur nachgerührt. Die Reaktionsmischung wird mit 150 ml Dichlormethan verdünnt und mit ges. Na₂CO₃-Lösung versetzt. Die wäßrige Phase wird noch mehrfach mit Dichlormethan extrahiert, die vereinigten organischen Phasen mit NaHSO₃-Lösung gewaschen und schließlich über Natriumsulfat getrocknet. Das Lösemittel wird im Vakuum entfernt und der Rückstand durch Chromatographie an Kieselgel (Eluens Dichlormethan/Methanol 70:1 bis 50 :1 (v/v)) gereinigt.
- Ausbeute:: 157 mg
- R_{f}:: 0,48 und 0,54 Laufmittel: CH₂Cl₂ / MeOH 10:1

### Beispiel 28: [N-(Ethoxycarboxymethyl)-N-(thymin-1-yl-acetyl)-2-aminoethyl]-2-cyanoethyl-(5'-(4-methoxytriryl-amino)-thymidin-3'-phosphat

Man löst 0,8 g (1,6 mmol) der Verbindung aus Beispiel 25 (aus Pentan gefällt und vorgetrocknet) in 40 ml Dichlormethan und versetzt unter Argon mit 1,2 mmol Bis(diisopropylammonium)-tetrazolid. Nach Zugabe von 2 eq. 2-Cyanoethoxy-bis-(diisopropylamino)-phosphin wird für zwei Stunden bei Raumtemperatur gerührt. Die Reaktionsmischung wird zwischen jeweils 40 ml Dichlormethan und ges. NaHCO₃-Lsg. verteilt, die wäßrige Phase noch dreimal mit Dichlormethan extrahiert und die vereinigten organischen Phasen mit NaCl-Lsg. gewaschen und anschließend über Natriumsulfat getrocknet. Das nach Verdampfen des Lösemittels resultierende Öl wird in wenig Dichlormethan gelöst und bei 0°C zu 400 ml Pentan gegeben. Die Lösung wird noch 30 Minuten bei dieser Temperatur belassen und schließlich der Niederschlag abfiltriert und getrocknet. Es resultieren 629 mg eines weißen Feststoffes, der im DC (EE/CH₂Cl₂/NEt₃ 45:45:10 (v/v/v)) zwei UV-absorbierende Punke für die beiden diastereomeren Phosphite zeigt. Das Rohprodukt (200 mg; 0,64 mmol) wird sofort weiter analog Beispiel 27 mit der Verbindung aus Beispiel 20 umgesetzt. Nach Chromatographie an Kieselgel mit CH₂Cl₂/MeOH-Gradienten (70:1 bis 30:1 (v/v)) erhält man das Produkt in Form eines weißen Feststoffes.
- Ausbeute:: 103 mg
- R_{f}:: 0,47 und 0,57 Laufmittel: CH₂Cl₂ / MeOH 10:1

## Patentansprüche

1. Verbindungen der allgemeinen Formel (I) 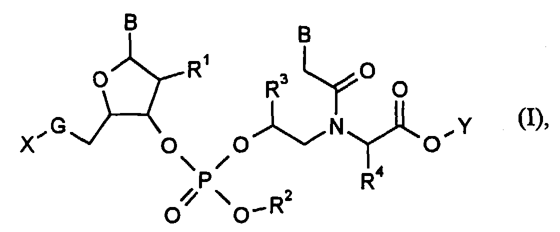 in welcher
R¹ für Wasserstoff, Hydroxy, C₁-C₁₈-Alkoxy, Halogen, Azido oder Amino steht,
R² für eine in der Nucleotidchemie übliche Phosphatschutzgruppe wie beispielsweise 2-Cyanoethyl steht,
R³ und R⁴ unabhängig voneinander für Wasserstoff, C₁-C₄-Alkyl oder gemeinsam für C₁-C₄-Alkandiyl wie beispielsweise -CH₂-, -(CH₂)₂-, -(CH₂)₃- oder -C(CH₃)₂- stehen,
B für alle natürlichen oder unnatürlichen Nukleobasen, wie z.B. Thymin, Uracil, Cytosin, Adenin, Guanin, Hypoxanthin oder durch chemische Modifikation von diesen abgeleitete Derivate oder halogenierte Vorstufen von diesen, gegebenenfalls an den Aminogruppen durch Schutzgruppen wie Acetyl, Trifluoracetyl, Trichloraceryl, Benzoyl, Phenylacetyl, Benzyloxycarbonyl (Z od. Cbz), tert.-Buryloxycarbonyl (Boc), Allyloxycarbonyl, (9-Fluorenyl)methoxycarbonyl (Fmoc) oder andere in der Peptid- und Nukleinsäurechemie übliche Schutzgruppen substituiert, steht,
G für -NH- oder -O- steht,
X für Wasserstoff oder eine beliebige aus der Peptidchemie bekannte Schutzgruppe wie beispielsweise Z, Fmoc oder 4-Methoxytrityl steht und
Y für Wasserstoff, C₁-C₆-Alkyl oder eine beliebige aus der Peptidchemie bekannte C-terminale Schutzgruppe wie beispielsweise Benzyl oder tert.-Butyl steht.

2. Verbindungen der allgemeinen Formel (I)
worin
R¹ für Wasserstoff, Hydroxy, C₁-C₆-Alkoxy, Fluor, Chlor, Brom, Azido oder Amino steht,
R² für eine in der Nucleotidchemie übliche Phosphatschutzgruppe steht,
R³ und R⁴ unabhängig voneinander für Wasserstoff, C₁-C₄-Alkyl oder gemeinsam für C₁-C₄-Alkandiyl bedeutet,
B für alle natürlichen Nukleobasen steht, wie z.B. Thymin, Uracil, Cytosin, Adenin, Guanin oder Hypoxanthin oder halogenierte Vorstufen von diesen, die gegebenenfalls an den Aminogruppen durch Schutzgruppen wie Acetyl, Trifluoracetyl, Trichloracetyl, Benzoyl, Phenylacetyl, Benzyloxycarbonyl, tert.-Buryloxycarbonyl, Allyloxycarbonyl, (9-Fluorenyl)methoxycarbonyl oder andere in der Peptid- und Nukleinsäurechemie übliche Schutzgruppen substituiert sind,
G für -NH- oder -O- steht,
X für Wasserstoff oder eine beliebige aus der Peptidchemie bekannte Schutzgruppe steht,
Y für Wasserstoff, C₁-C₄-Alklyl oder eine beliebige aus der Peptidchemie bekannte C-terminale Schutzgruppe steht.

3. Verbindungen der allgemeinen Formel (I) gemäß Anspruch 1, worin
R¹ für Wasserstoff, Hydroxy, Methoxy, Ethoxy, Fluor, Chlor, Azido oder Amino steht,
R² für 2-Cyanoethyl steht,
R³ und R⁴ unabhängig voneinander für Wasserstoff, Methyl, Ethyl, Isopropyl oder gemeinsam für -CH₂-, -(CH₂)₂-, -(CH₂)₃- oder -C(CH₃)₂- stehen,
B für alle natürlichen Nuldeobasen, wie z.B. Thymin, Uracil, Cytosin, Adenin, Guanin oder Hypoxanthin oder halogenierte Vorstufen von diesen, die gegebenenfalls an den Aminogruppen durch Schutzgruppen wie Acetyl, Trifluoracetyl, Trichloracetyl, Benzoyl, Phenylacetyl, Benzloxycarbonyl, tert.-Butyloxycarbonyl, Allyloxycarbonyl, (9-Fluorenyl)methoxycarbonyl oder andere in der Peptid- und Nukleinsäurechemie übliche Schutzgruppen substituiert sind, steht,
G für -NH- oder -O- steht,
X für Wasserstoff oder eine beliebige aus der Peptidchemie bekannte Schutzgruppe steht,
Y für Wasserstoff, Methyl, Ethyl, n-Propyl, Isopropyl, n-Butyl, Isobutyl, sec.-Butyl, tert.-Butyl oder Benzyl steht.

4. Verbindungen der allgemeinen Formel (II) 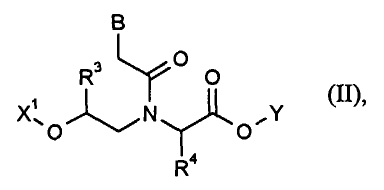 in welcher
R³, R⁴ und B die bei der Beschreibung der Verbindungen der Formel (I) angegebenen Bedeutungen haben,
X¹ für Wasserstoff oder eine aus der Peptidchemie bekannte säurelabile Schutzgruppe wie beispielsweise durch Alkyl und/oder Phenyl substituiertes Silyl, Z, Fmoc oder 4-Methoxytrityl steht, wobei für den Fall, daß R³ und R⁴ gleichzeitig jeweils für Wasserstoff stehen, gegebenenfalls substituiertes Alkoxycarbonyl und Trityl ausgenommen sind und
Y für C₁-C₆-Alkyl oder eine beliebige aus der Peptidchemie bekannte C-terminale Schutzgruppe wie beispielsweise Benzyl oder tert.-Butyl steht.
